# EUROPEAN PATENT SPECIFICATION

(11) **EP 0 308 096 B1**
(45) Date of publication and mention of the grant of the patent: **03.02.1993**
(21) Application number: 88307950.1
(22) Date of filing: 26.08.1988
(51) Int. Cl.: C07C 6/12

(54) **Toluene disproportionation**
Verfahren zur Disproportionierung von Toluol
Procédé pour le disproportionnement de toluène

(30) Priority: 02.09.1987 US 92842
(43) Date of publication of application: 22.03.1989
(73) Proprietor: MOBIL OIL CORPORATION, New York New York 10017 (US)
(72) Inventor: Absil, Robert Peter, Mantua New Jersey 08051 (US); Han, Scott, Lawrenceville New Jersey 08648 (US); Marler, David Owen, Deptford New Jersey 08096 (US); Shihabi, David Said, Pennington New Jersey 08534 (US)
(74) Representative: Colmer, Stephen Gary

(56) References cited:
- US-A- 4 052 476

## Description

This invention is directed to a process for effecting vapor-phase disproportionation of toluene.

It is known from, for example, U.S. Patent No. 4,052,476 to effect vapor phase disproportionation of toluene by contacting toluene with a zeolite having a Constraint Index of 1 - 12, preferably ZSM-5, at a temperature of 650 - 1100°F (340 - 600°C), a hydrogen to hydrocarbon mole ratio of 0-4, a pressure of atmospheric to 1000 psig (7000 kPa) and a WHSV of 1-20.

Catalyst aging is, however, a recurrent problem with toluene disproportionation processes and there is therefore a continuing need to reduce catalyst aging. The present invention addresses this problem.

Accordingly, the invention resides in a process for the effecting vapor phase disproportionation of toluene comprising contacting toluene under conversion conditions with a catalyst composition comprising a crystalling zeolite having a silica/alumina mole ratio greater than 12 and less than 55, a Constraint Index of 1 to 12 and a diffusion rate constant of less than 150 sec⁻¹.

The diffusion rate constant of a particular crystalline zeolite is defined as D/r² x 10⁶ wherein D is the diffusion coefficient (cm²/sec) and r is the crystal radius (cm). The required diffusion parameters can be derived from sorption measurements provided the assumption is made that the plane sheet model describes the diffusion process. Thus for the given sorbate loading Q, the value Q/Q_{∞}, where Q_{∞} is the equilibrium sorbate loading, is directly proportionate to (Dt/r²)^{1/2} where t is the time (sec) required to reach the sorbate loading Q. Graphical solutions for the plane sheet model given by J. Crank in "The Mathmatics of Diffusion", Oxford University Press, Ely House, London, 1967, are tabulated below:

| Q/Q_{∞} | (Dt/r²)^{1/2} |
|---|---|
| 0.05 | 0.044 |
| 0.10 | 0.088 |
| 0.20 | 0.173 |
| 0.30 | 0.267 |
| 0.40 | 0.353 |

The process of the present invention employs a catalyst comprising a zeolite having a Constraint Index of 1 to 12 (see U.S. Patent No. 4,016,218) and a silica/alumina mole ratio less than 55, preferably from 20 to less than 55, more preferably from 20 to 40. The zeolite also has a diffusion rate constant (D/r² x 10⁶) of less than about 150 sec ⁻¹, and preferably less than about 120 sec⁻¹. Suitable zeolites include ZSM-5, ZSM-11, ZSM-12, ZSM-22, ZSM-23, ZSM-35, ZSM-48, ZSM-50 and zeolite beta with ZSM-5 being preferred. ZSM-5 is described in U.S. Patent 3,702,886; ZSM-11 is described in U.S. Patent 3,709,979; ZSM-12 is described in U.S. Patent 3,832,449; ZSM-22 is described in Canadian Patent No. 1,210,747; ZSM-23 is described in U.S. Patent 4,076,842; ZSM-35 is described in U.S. Patent 4,016,245; ZSM-48 is described in U.S. Patent 4,397,827; ZSM-50 is described in U.S. Patent 4,640,849; and zeolite beta is described in U.S. Patent 3,308,069.

| Constraint Index (CI) values for some typical materials are: | | |
|---|---|---|
| | CI | (at test temperature) |
| ZSM-4 | 0.5 | (316°C) |
| ZSM-5 | 6-8.3 | (371°C - 316°C) |
| ZSM-11 | 5-8.7 | (371°C - 316°C) |
| ZSM-12 | 2.3 | (316°C) |
| ZSM-20 | 0.5 | (371°C) |
| ZSM-22 | 7.3 | (427°C) |
| ZSM-23 | 9.1 | (427°C) |
| ZSM-34 | 50 | (371°C) |
| ZSM-35 | 4.5 | (454°C) |
| ZSM-38 | 2 | (510°C) |
| ZSM-48 | 3.5 | (538°C) |
| ZSM-50 | 2.1 | (427°C) |
| TMA Offretite | 3.7 | (316°C) |
| TEA Mordenite | 0.4 | (316°C) |
| Clinoptilolite | 3.4 | (510°C) |
| Mordenite | 0.5 | (316°C) |
| REY | 0.4 | (316°C) |
| Amorphous Silica-alumina | 0.6 | (538°C) |
| Dealuminized Y | 0.5 | (510°C) |
| Erionite | 38 | (316°C) |
| Zeolite Beta | 0.6-2.0 | (316°C-399°C) |

It will be noted that the Constraint Index values for some of the above zeolite vary with temperature. To be suitable for use in the present invention, a zeolite must have a Constraint Index value within the range 1-12 when tested at some temperature in the range 290 - 538°C.

For the disproportionation process of this invention the zeolite catalyst may be employed in combination with a support or binder material such as, for example, a porous inorganic oxide support or a clay binder. Non-limiting examples of such binder materials include alumina, zirconia, silica, magnesia, thoria, titania, boria and combinations thereof, generally in the form of dried inorganic oxide gels and gelatinous precipitates. Suitable clay materials include, by way of example, bentonite and kieselguhr. The relative proportion of suitable crystalline molecular sieve of the total composition of catalyst and binder or support may vary widely with the zeolite content ranging from between 30 to 90 percent by weight and more usually in the range of 50 to 80 percent by weight of the composition. The composition may be in the form of an extrudate, beads or fluidizable microspheres.

The improved process of this invention is conducted such that disproportionation of toluene is carried out in the vapor-phase by contact in a reaction zone, such as, for example, a fixed bed of catalyst composition, under disproportionation effective conditions, said catalyst composition being characterized as comprising the above-defined molecular sieve, preferably which has been hydrogen, hydrogen precursor and/or non-noble Group VIII metal exchanged and/or thermally treated. The effluent is separated and distilled to remove desired product, such as benzene and xylene, and unreacted reactant, i.e. toluene, is recycled for further reaction.

By the present improved process toluene is converted to aromatic concentrates of high value, e.g. xylene and benzene. This process may be conducted in either batch or fluid bed operation with attendant benefits of either operation readily obtainable.

In the process of this invention, the toluene charge is preferably dried in a manner which will minimize the water entering the reaction employed. Means known in the art suitable for drying the toluene charge to the present process are numerous, including percolation through silica gel, activated alumina, molecular sieves or other suitable substance or use of liquid charge dryers.

In a typical embodiment of the present process, optimum toluene conversion is found to be from about 40 weight percent to about 50 weight percent. Yield of C₅⁻ products and ring losses in such an embodiment appear to increase at conversion above about 40 percent and xylene yields begin to decrease when toluene conversion exceeds about 50 weight percent.

Suitable conditions for the process of the invention include a temperature of 600°F to 1100°F (316 - 593°C), preferably 650°F to about 1000°F (343 - 540°C) at a pressure of atmospheric to 1000 psig (7000 kPa), more preferably 50 to 1000 psig (450 - 7000 kPa). The hydrogen to hydrocarbon mole ratio may be from 0 (no added hydrogen) to 10, with a preferred range of from 0 to 3. A particularly preferred range of hydrogen to hydrocarbon mole ratio will be from 0 to 2.

The invention will now be described with reference to the Examples in which all parts are given by weight, unless otherwise stated. In the Examples, when Alpha Value is examined, it is noted that the Alpha Value is an approximate indication of the catalytic cracking activity of the catalyst compared to a standard catalyst and gives the relative rate constant (rate of normal hexane conversion per volume of catalyst per unit time). It is based on the activity of a silica-alumina cracking catalyst taken as an Alpha of 1 (Rate Constant = 0.016 sec ⁻¹). The Alpha Test is described in U.S. Patent 3,354,078 and in The Journal of Catalysis, Vol. IV, pp. 522-529 (August 1965).

To assist in understanding the Examples, the attached drawing is also provided which is a graph of temperature against days on stream in a toluene disproportionation process employing Catalysts A - E described in the Examples.

### Example 1

Five separate ZSM-5 samples were prepared as follows:

### Molecular Sieve A

6.4 parts of water were mixed with 11.7 parts 50% NaOH, 10.6 parts Al₂(SO₄)₃ 14H₂O and 71.4 parts amorphous silica (46.5% solids), prepared by the neutralization of sodium silicate with sulfuric acid. The reaction mixture had a composition, in mole ratios of:
SiO₂/Al₂O₃ = 30
H₂O/SiO₂ = 5.76
OH⁻/SiO₂ = 0.072
OH⁻/H₂O = 0.013

The reaction mixture was then heated to 350°F (177°C) and stirred in an autoclave at that temperature for crystallization. After full crystallinity was achieved, the resulting crystals were separated from remaining liquid by filtration, washed with water and dried.

### Molecular Sieve B

7.3 parts of water were mixed with 12.8 parts 50% NaOH, 10.1 parts Al₂(SO₄)₃ · 14 H₂O, 1.6 parts ZSM-5 seeds and 68.2 parts amorphous silica (47.6% solids) prepared by the neutralization of sodium silicate with sulfuric acid. The reaction mixture had a composition, in mole ratios, of:
SiO₂/Al₂O₃ = 32
H₂O/SiO₂ = 5.45
OH⁻/SiO₂ = 0.105
OH⁻/H₂O = 0.0192

The reaction mixture was then heated directly to 220°F (104°C) and stirred in an autoclave at that temperature for crystallization. After full crystallinity was achieved, the resulting crystals were separated from remaining liquid by filtration, washed with water and dried.

### Molecular Sieve C

3.1 parts of n-propylamine were added to a mixture containing 1.1 parts sodium chloride, 0.2 parts ZSM-5 seeds, 0.2 parts dispersant (mixture of polymerized aryl and substituted benzoid alkyl sulfonic acids), 2.6 parts Al₂ (SO₄)₃ · 14H₂O, 7.0 parts 50% NaOH, 25.8 parts HiSil 233 (a precipitated hydrated SiO₂ containing about 6 wt.% free H₂O and about 4.5 wt.% bound H₂O of hydration and having an ultimate particle size of about 0.02 micron) and 59.9 parts water. The reaction mixture had a composition, in mole ratios, of:
SiO₂/Al₂O₃ = 65
H₂O/SiO₂ = 9.92
OH⁻/SiO₂ = 0.163
N/Al₂O₃ = 9.2
OH⁻/H₂O = 0.0165
wherein N is the n-propylamine. In the above ratios, the hydroxide concentration is based on only inorganic sources.

The reaction mixture was then heated directly to 220°F (104°C) and stirred in an autoclave at that temperature for crystallization. After full crystallinity was achieved, the resulting crystals were separated from remaining liquid by filtration, washed with water, exchanged with NH₄NO₃ and dried.

### Molecular Sieve D

3.1 parts of n-propylamine were added to a mixture containing 1.1 parts sodium chloride, 0.2 parts ZSM-5 seeds, 0.2 parts dispersant (mixture of polymerized aryl and substituted benzoid alkyl sulfonic acids), 2.6 parts Al₂(SO₄)₃ · 14 H₂O, 7.0 parts 50% NaOH, 25.8 parts HiSil 233 and 59.9 parts water. The reaction mixture had a composition, in mole ratios, of:
SiO₂/Al₂O₃ = 65
H₂O/SiO₂ = 9.92
OH⁻/SiO₂ = 0.163
N/Al₂O₃ = 9.2
OH⁻/H₂O = 0.0165
wherein N is the n-propylamine. In the above ratios, the hydroxide concentration is based on only inorganic sources.

The reaction mixture was then heated directly to 320°F (160°C) and stirred in an autoclave at that temperature for crystallization. After full crystallinity was achieved, the resulting crystals were separated from remaining liquid by filtration, washed with water, exchanged with NH₄NO₃ and dried.

### Molecular Sieve E

1.0 parts of water was mixed with 7.0 parts 100% NaOH, 10.8 parts Al₂(SO₄)₃ · 14 H₂O, 75.6 parts amorphous silica (45.2% solids) prepared by the neutralization of sodium silicate with sulfuric acid, and 5.5 parts ZSM-5 seeds (33% solids). The reaction mixture had a composition, in mole ratios, of:
SiO₂/Al₂O₃ = 31
H₂O/SiO₂ = 4.95
OH⁻/SiO₂ = 0.109
OH⁻/H₂O = 0.0219

The reaction mixture was then heated to 220°F (104°C) and stirred in an autoclave at that temperature for crystallization. After full crystallinity was achieved, the resulting crystals were separated from remaining liquid by filtration, washed with water and dried.

The above molecular sieve materials were evaluated for diffusion rate constants, composition, e.g. alumina, silica and sodium contents, surface area, particle density, pore volume and Alpha Value. Results of these evaluations are listed in Table I below.

**TABLE I**

| Molecular Sieve | A | B | C | D | E |
|---|---|---|---|---|---|
| SiO₂/Al₂O₃, mole ratio | 26 | 26 | 55 | 55 | 26 |
| Na,ppm | 135 | 120 | 450 | 280 | -- |
| Diffusion rate (D/r² x 10⁶),sec⁻¹ | <150 | >150 | >150 | <150 | >150 |
| Surface area,m²/g | 325 | 317 | 349 | 265 | -- |
| Particle density,g/cc | 0.87 | 1.01 | 0.88 | 0.93 | -- |
| Pore volume,cc/g | 0.77 | 0.61 | 0.76 | 0.70 | -- |
| Alpha Value | 650 | 710 | 350 | 290 | 427 |

### Example 2

The molecular sieves A - E of Example 1 were each composited with an alumina binder and made into extrudates Catalysts A - E respectively, each catalyst comprising 65 wt.% zeolite and 35 wt.% alumina.

Each catalyst was then diluted and evaluated for toluene disproportionation in identical reactors under identical conditions. 2.3 g Catalyst A was diluted with 4.5 g inert sand, whereas 1.1 g of each of Catalysts B, C and D with 1.0 cc inert Vycor quartz chips. The reactions were conducted in 3/8-inch (0.95 cm) outside diameter stainless steel reactors and the reaction conditions were 600 psig (4240 kPa), 4.0 hr⁻¹ weight hourly space velocity (based on molecular sieve) and a hydrogen/hydrocarbon mole ratio of 2, with the temperature being adjusted to maintain a target toluene conversion of 48 ± 1 wt.%. The toluene feedstock was dried for each reaction by percolation through activated alumina.

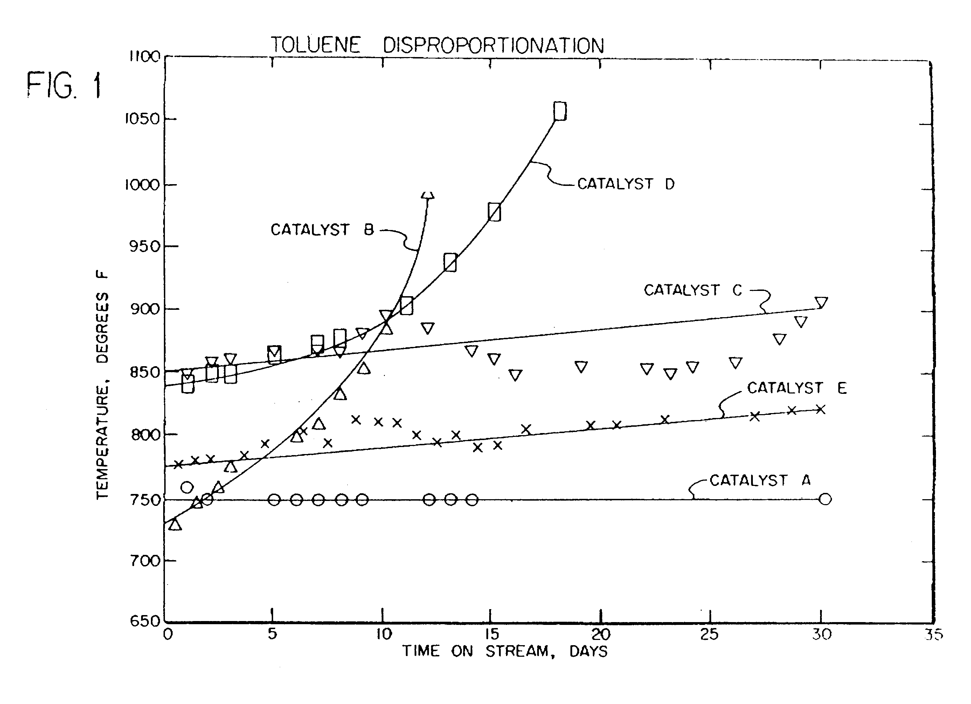

Liquid and gas products from the reactions were analyzed by conventional chromatography. Run data are presented in Figure 1, which is a plot of reaction temperature in °F versus time on stream in days for each of the Example 2 disproportionation runs.

It is noted that for Catalyst A the start-of-cycle temperature was 750°F (400°C), which was maintained throughout the run. For Catalyst B, the initial start-of-cycle temperature was 730°F (388°C). Since Catalyst B aged rapidly, the temperature was corrected for the target conversion when necessary by using a factor of 3 wt.% toluene conversion/10°F (5°C). The same aging correction factor was used for the runs with catalysts C and D. For Catalyst C, the start-of-cycle temprature was 849°F (454°C); and for Catalyst D, 847°F (453°C). Catalyst E reached the 48% target conversion at 775°F (413°C) initially and aged to 802°F (428°C).

From the data plotted in Figure 1, it is observed that Catalyst A showed no appreciable aging ( < 0.1 °C/day) over a 30 day cycle. Catalyst E, with a silica/alumina mole ratio of 26, but a diffusion rate of greater than 150 sec⁻¹, aged 0.5°C/day over the 30 day cycle. Catalyst C, with a silica/alumina mole ratio of 55 and a diffusion rate constant of greater than 150 sec⁻¹, aged over 28°C (50°F) in the same 30 day time period, giving an aging rate of > 0.5°C/day. Catalyst B, with the diffusion rate constant greater than 150 sec ⁻¹, and Catalyst D, with a silica/alumina mole ratio of 55, both showed severe aging at a rate of > 5°C/day.

## Claims

1. A process for effecting vapor phase disproportionation of toluene comprising contacting toluene under conversion conditions with a catalyst composition comprising a crystalline zeolite having a silica/alumina mole ratio greater than 12 and less than 55, a Constraint Index of 1 to 12 and a diffusion rate constant of less than 150 sec ⁻¹.

2. The process of claim 1 wherein said zeolite has a silica/alumina mole ratio of from about 20 to about 40.

3. The process of claim 1 or claim 2 wherein said zeolite has a diffusion rate constant of less than about 120 sec ⁻¹.

4. The process of any preceding claim wherein said zeolite is ZSM-5, ZSM-11, ZSM-12, ZSM-22, ZSM-23, ZSM-35, ZSM-48, ZSM-50 or Beta.

5. The process of any preceding claim wherein the zeolite is ZSM-5.

6. The process of any preceding claim wherein said conversion conditions include a temperature of 316 - 593°C (600 to 1100°F), a pressure of atmospheric to 7000 kPa (1000 psig), a hydrogen/hydrocarbon mole ratio of 0 to 10 and a weight hourly space velocity, based upon weight of active catalyst component, of 0.1 to 30 hr ⁻¹.

7. The process of any preceding claim wherein said conversion conditions include a temperature of 343 - 540°C (650 - 1000°F), a pressure of 450 to 7000 kPa (50 to 1000 psig) and a hydrogen/hydrocarbon mole ratio of 0 to 3.

## Patentansprüche

1. Verfahren zur Durchführung der Disproportionierung von Toluol in der Dampfphase, das den Kontakt von Toluol bei Umwandlungsbedingungen mit einer Katalysatorzusammensetzung umfaßt, die einen kristallinen Zeolith mit einem Siliciumdioxid/Aluminiumoxid-Molverhältnis von mehr als 12 und weniger als 55, einem Zwangsindex von 1 bis 12 und eine Diffusionsgeschwindigkeitskonstante von weniger als 150 s⁻¹ umfaßt.

2. Verfahren nach Anspruch 1, worin der Zeolith ein Siliciumdioxid/Aluminiumoxid-Molverhältnis von etwa 20 bis etwa 40 aufweist.

3. Verfahren nach Anspruch 1 oder 2, worin der Zeolith eine Diffusionsgeschwindigkeitskonstante von weniger als etwa 120 s⁻¹ aufweist.

4. Verfahren nach einem der vorstehenden Ansprüche, worin der Zeolith ZSM-5, ZSM-11, ZSM-12, ZSM-22, ZSM-23, ZSM-35, ZSM-48, ZSM-50 oder Beta ist.

5. Verfahren nach einem der vorstehenden Ansprüche, worin der Zeolith ZSM-5 ist.

6. Verfahren nach einem der vorstehenden Ansprüche, worin die Umwandlungsbedingungen eine Temperatur von 316-593°C (600-1100°F), einen Druck von atmosphärischem Druck bis 7000 kPa (1000 psig), ein Wasserstoff/Kohlenwasserstoff-Molverhältnis von 0 bis 10 und eine stündliche Gewichts-Raum-Geschwindigkeit auf der Basis des Gewichtes der aktiven Katalysatorkomponente von 0,1 bis 30 h⁻¹ umfassen.

7. Verfahren nach einem der vorstehenden Ansprüche, worin die Umwandlungsbedingungen eine Temperatur von 343-540°C (650-1000°F), einen Druck von 450 bis 7000 kPa (50 bis 1000 psig), ein Wasserstoff/Kohlenwasserstoff-Molverhältnis von 0 bis 3 umfassen.

## Revendications

1. Un procédé de disproportionnement du toluène en phase vapeur consistant à mettre le toluène, dans des conditions de conversion, au contact d'une composition de catalyseur comprenant des zéolites cristallines dont le rapport molaire silice/alumine est supérieur à 12 et inférieur à 55, l'indice de contrainte est compris entre 1 et 12 et la constante de taux de diffusion est inférieure à 150 s⁻¹.

2. Le procédé selon la revendication 1, caractérisé en ce que le rapport molaire silice/alumine de cette zéolite est compris entre environ 20 et environ 40.

3. Le procédé selon la revendication 2, caractérisé en ce que la constante de taux de diffusion de cette zéolite est inférieur à environ 120 s⁻¹.

4. Le procédé selon l'une quelconque des revendications précédentes, caractérisé en ce que cette zéolite est la ZSM-5, ZSM-11, ZSM-12, ZSM-22, ZSM-23, ZSM-35, ZSM-48, ZSM-50 ou la zéolite bêta.

5. Le procédé selon l'une quelconque des revendications précédentes, caractérisé en ce que la zéolite est la ZSM-5.

6. Le procédé selon l'une quelconque des revendications précédentes, caractérisé en ce que ces conditions de conversion comprennent une température de 316 à 593°C (600 à 1100°F), une pression comprise entre la pression atmosphérique et 7000 kPa (1000 psig), un rapport molaire hydrogène/hydrocarbure de 0 à 10 et une vitesse spatiale horaire pondérale de 0,1 à 30 h⁻¹ sur la base du poids du composant de catalyseur actif.

7. Le procédé selon l'une quelconque des revendications précédentes, caractérisé en ce que ces conditions de conversion comprennent une température de 343 à 540°C (650 à 1000°F), une pression de 450 à 7000 kPa ( 50 à 1000 psig) et un rapport molaire hydrogène/hydrocarbure de 0 à 3.
